# EUROPEAN PATENT APPLICATION

(11) **EP 4 737 435 A1**
(43) Date of publication of application: **06.05.2026**
(21) Application number: 24831410.6
(22) Date of filing: 07.05.2024
(51) Int. Cl.: C07C 209/48, C07C 211/27, C07B 61/00

(54) **METHOD FOR PRODUCING XYLYLENEDIAMINE**

(30) Priority: 30.06.2023 JP 2023108290
(71) Applicant: MITSUBISHI GAS CHEMICAL COMPANY, INC., Chiyoda-ku Tokyo 100-8324 (JP)
(72) Inventor: YOKOO, Kazuma, Kurashiki-shi, Okayama 712-8525 (JP); IBI, Yukiya, Kurashiki-shi, Okayama 712-8525 (JP); KUMANO, Tatsuyuki, Kurashiki-shi, Okayama 712-8525 (JP)
(74) Representative: Müller-Boré & Partner Patentanwälte PartG mbB
(86) International application number: PCT/JP2024/016985
(87) International publication number: WO 2025/004537

(57) **Abstract**

Provided is a xylylenediamine production method including:
a first hydrogenation step of supplying dicyanobenzene and hydrogen-containing gas G1 in the presence of a catalyst having hydrogenation ability to a reactor to obtain xylylenediamine by hydrogenation of the dicyanobenzene;
a treatment step of interrupting the hydrogenation and supplying hydrogen-containing gas G2 comprising recycled gas to the reactor to bring the catalyst that has undergone the first hydrogenation step into contact with the hydrogen-containing gas G2; and
a second hydrogenation step of resuming the hydrogenation in the presence of the catalyst that has undergone the treatment step, wherein
the recycled gas is obtained by removing at least a part of an organic compound from exhaust gas emitted from the reactor through the contact.

## Description

### Technical Field

The present invention relates to a xylylenediamine production method.

### Background Art

Xylylenediamine is useful as a starting material for polyamide resins, curing agents, and the like, and as an intermediate material for isocyanate resins and the like. A method for producing xylylenediamine typically includes a method of hydrogenating dicyanobenzene in the presence of a catalyst. As a specific example thereof, Patent Literature 1, for example, proposes that a catalyst whose activity has been reduced by use in the hydrogenation reaction of dicyanobenzene is subjected to regeneration treatment by the following steps (1) and (2) and then reused as a catalyst in a reaction system:
(1) the step of bringing the catalyst into contact with hydrogen-containing gas at a temperature that falls within the range of 140 to 200°C and has an average temperature of 180°C or lower for 1 hour or longer; and
(2) the step of further bringing the catalyst thus treated by the contact into contact with hydrogen-containing gas at a temperature in the range of 200 to 500°C.

### Citation List

### Patent Literature

Patent Literature 1: Japanese Patent No. 4561063

### Summary of Invention

### Technical Problem

The method described in Patent Literature 1 permits drastic reduction in catalyst cost and is therefore advantageous, because a catalyst with reduced activity can be reused. On the other hand, in Patent Literature 1, sufficient studies have hardly been made on the composition of gas for treating the catalyst, for example. Thus, the method still has room for improvement from the viewpoint of production efficiency.

The present invention has been made in light of these problems, and an object of the present invention is to provide a xylylenediamine production method that enables a catalyst to be used for a long period in hydrogenation of dicyanobenzene and is also excellent in production efficiency.

### Solution to Problem

The present inventors have conducted diligent studies to attain the object and consequently completed the present invention by finding that the object can be attained, for example, by treating exhaust gas in the regeneration treatment of a catalyst and then bringing the gas into contact with the catalyst after use.

Specifically, the present invention encompasses the following aspects.
[1] A xylylenediamine production method comprising:
   a first hydrogenation step of supplying dicyanobenzene and hydrogen-containing gas G1 in the presence of a catalyst having hydrogenation ability to a reactor to obtain xylylenediamine by hydrogenation of the dicyanobenzene;
   a treatment step of interrupting the hydrogenation and supplying hydrogen-containing gas G2 comprising recycled gas to the reactor to bring the catalyst that has undergone the first hydrogenation step into contact with the hydrogen-containing gas G2; and
   a second hydrogenation step of resuming the hydrogenation in the presence of the catalyst that has undergone the treatment step, wherein
   the recycled gas is obtained by removing at least a part of an organic compound from exhaust gas emitted from the reactor through the contact.
[2] The xylylenediamine production method according to [1], wherein the recycled gas is obtained by removing at least a part of the organic compound from the exhaust gas using a gas-liquid separator.
[3] The xylylenediamine production method according to [2], wherein a supply temperature of the exhaust gas to the gas-liquid separator is 5°C to 80°C.
[4] The xylylenediamine production method according to any of [1] to [3], wherein in the treatment step, a contact temperature between the hydrogen-containing gas G2 and the catalyst that has undergone the first hydrogenation step is 200°C to 500°C.
[5] The xylylenediamine production method according to any of [1] to [4], wherein a hydrogen concentration of the hydrogen-containing gas G2 is 1 vol% or more.
[6] The xylylenediamine production method according to any of [1] to [5], wherein in the treatment step, at least a part of the catalyst is washed with a solvent before the supply of the hydrogen-containing gas G2 to the reactor.
[7] The xylylenediamine production method according to any of [1] to [6], wherein the reactor is a fixed-bed reactor.
[8] The xylylenediamine production method according to any of [1] to [7], wherein the catalyst comprises nickel and/or cobalt.

### Advantageous Effect of Invention

The present invention can provide a xylylenediamine production method that enables a catalyst to be used for a long period in hydrogenation of dicyanobenzene and is also excellent in production efficiency.

### Description of Embodiments

Hereinafter, the embodiment for carrying out the present invention (hereinafter, referred to as the "present embodiment") will be described in detail. The present embodiment given below is an illustration for describing the present invention and does not intend to limit the present invention to the contents described below. The present invention can be carried out through appropriate changes or modifications made without departing from the spirit of the present invention.

### <Xylylenediamine production method>

The xylylenediamine production method of the present embodiment (hereinafter, also referred to as the "production method of the present embodiment") comprises: a first hydrogenation step of supplying dicyanobenzene and hydrogen-containing gas G1 in the presence of a catalyst having hydrogenation ability (hereinafter, also simply referred to as a "catalyst") to a reactor to obtain xylylenediamine by hydrogenation of the dicyanobenzene; a treatment step of interrupting the hydrogenation and supplying hydrogen-containing gas G2 comprising recycled gas to the reactor to bring the catalyst that has undergone the first hydrogenation step into contact with the hydrogen-containing gas G2; and a second hydrogenation step of resuming the hydrogenation in the presence of the catalyst that has undergone the treatment step, wherein the recycled gas is obtained by removing at least a part of an organic compound from exhaust gas emitted from the reactor through the contact. The production method of the present embodiment thus configured enables a catalyst to be used for a long period in hydrogenation of dicyanobenzene and is also excellent in production efficiency.

### (First hydrogenation step)

In the first hydrogenation step, dicyanobenzene and hydrogen-containing gas G1 are supplied in the presence of a catalyst having hydrogenation ability to a reactor to obtain xylylenediamine by hydrogenation of the dicyanobenzene. A method or reaction conditions for this hydrogenation (hydrogenation reaction) are not particularly limited, and various known methods or conditions can be adopted. Hereinafter, typical examples thereof will be described.

The dicyanobenzene means a compound obtained by replacing at least two hydrogen atoms of benzene with nitrile groups. Examples thereof include isophthalonitrile and terephthalonitrile. A compound substituted by these nitrile groups and further substituted by a halogen atom such as fluorine or chlorine, an alkyl group such as a methyl group or an ethyl group, or an alkoxy group such as a methoxy group or an ethoxy group, for example, 2-chloroterephthalonitrile, 5-methylisophthalonitrile, or 4-methylisophthalonitrile, can also be used. Such a compound is subjected to hydrogenation reaction to obtain corresponding xylylenediamine.

In the present embodiment, the hydrogenation reaction is preferably performed under liquid phase conditions. In this respect, a reaction solvent is preferably used. The reaction solvent is not particularly limited, and various solvents stable under hydrogenation reaction conditions can be used. Specific examples thereof include hydrocarbon-based solvents such as toluene, xylene, and trimethylbenzene, ether-based solvents such as tetrahydrofuran and dioxane, lower aliphatic amide-based solvents such as dimethylformamide and dimethylacetamide, alcohol-based solvents such as methanol, ethanol, and propanol, and ammonia. Two or more types may be selected from these solvents and used in combination. Ammonia is preferably selected as a part of the solvent because use of the ammonia can enhance a xylylenediamine yield. The amount of the solvent used is not particularly limited and is preferably 1 to 99 parts by mass, more preferably 1.5 to 99 parts by mass, based on 1 part by mass of the dicyanobenzene.

The hydrogen-containing gas G1 for use in the hydrogenation reaction of the dicyanobenzene may contain, in addition to hydrogen, impurities that are not involved in the reaction, for example, methane and nitrogen. The hydrogen concentration is preferably 50 mol% or more from the viewpoint of decreasing the concentration of the impurities and thereby easily securing a necessary hydrogen partial pressure and avoiding elevation in reaction total pressure.

A known hydrogenation catalyst such as a supported or non-supported metal catalyst or a Raney catalyst can be used as the catalyst having hydrogenation ability according to the present embodiment. A catalyst containing at least one metal selected from nickel, cobalt, palladium, ruthenium, and rhodium as an active metal component is preferred. Among them, a catalyst containing nickel and/or cobalt is more preferred. In the case of a supported catalyst, examples of the support used include alumina, silica, titania, and zirconia. The catalyst can be modified, if necessary, by the addition of at least one component selected from the group consisting of Li, Na, K, Rb, Cs, Be, Ca, Ba, Ti, Cu, Cr, Zn, Mn, Mg, Fe, Ga, Ge, Nb, Ir, Pt, Bi, Al, Si, In, Sr, Ce, and Mo.

In the present embodiment, the catalyst may be subjected to reduction treatment (activation treatment) before being used in the hydrogenation, from the viewpoint of drawing the performance of the catalyst. For this reduction treatment, various known conditions can be adopted.

In the present embodiment, an additive may be added in the hydrogenation reaction for the purpose of accelerating the reaction and improving a yield, for example. Examples of the additive include hydroxides and alcoholates of alkali metals or alkaline earth metals and specifically include lithium hydroxide, sodium hydroxide, and potassium hydroxide.

The format of the hydrogenation reaction may be any of a fixed-bed format and a suspended-bed format and may be any of a batch format and a continuous format. A reactor adapted therefor can be used. In the present embodiment, a continuous-flow fixed-bed format is convenient and thus preferred, and a reactor adapted therefor is preferably used. Specifically, in the present embodiment, the reactor is preferably a fixed-bed reactor.

The reaction temperature of the hydrogenation reaction is preferably 20 to 250°C, more preferably 20 to 200°C, and the hydrogen pressure is preferably 0.5 to 30 MPa, more preferably 1 to 20 MPa. The reaction temperature of the hydrogenation reaction can be identified as a catalyst layer temperature during reaction.

In the case of carrying out the hydrogenation in a suspended bed, the amount of the catalyst used is preferably 0.1 to 200 parts by mass based on 100 parts by mass of the starting material dicyanobenzene. In the case of carrying out the hydrogenation in a fixed bed, the starting material dicyanobenzene is preferably supplied at a rate of 0.01 to 1000 parts by mass/hr based on 100 parts by mass of the catalyst.

In the hydrogenation reaction, it is preferred to elevate the degree of progression of the hydrogenation reaction of nitrile groups to aminomethyl groups, and it is preferred to select reaction conditions under which the concentrations of dicyanobenzene and cyanobenzylamine in a liquid obtained after the hydrogenation reaction are kept low, from the viewpoint of efficiently producing xylylenediamine. Specifically, the amount of the cyanobenzylamine in a reaction product obtained after the hydrogenation reaction is preferably kept at 5.0% by mass or less, more preferably at 1.0% by mass or less, further preferably at 0.2% by mass or less. A product obtained by removing the solvent from the reaction liquid discharged from the reactor preferably attains, as the reaction product, the range described above. The conversion rate of the dicyanobenzene is preferably 99.50% or more, more preferably 99.90% or more, further preferably 99.95% or more.

### (Treatment step)

In the treatment step, the hydrogenation is interrupted, and hydrogen-containing gas G2 comprising recycled gas is supplied to the reactor to bring the catalyst that has undergone the first hydrogenation step into contact with the hydrogen-containing gas G2.

In the present embodiment, when the catalyst is subjected to hydrogenation, its activity is reduced over time. Specifically, in the present embodiment, the catalyst immediately after the first hydrogenation step is treated as having reduced activity compared to the catalyst immediately before application to the first hydrogenation step. In the present embodiment, the treatment step can be carried out so that the catalyst that has undergone the first hydrogenation step comes into contact with the hydrogen-containing gas G2, thereby reactivating the catalyst (in the present specification, also simply referred to as "regeneration" or "regeneration treatment"). Not only can desired reaction results be achieved in the subsequent second hydrogenation step, but production efficiency is excellent. The present inventors have deduced the reason for this as described below, though not intending to limit the reason to the following contents.

As mentioned above, the catalyst according to the present embodiment exhibits reduced activity through the first hydrogenation step. Thus, for continuing xylylenediamine production without deteriorating reaction results, it is important to carry out regeneration treatment of the catalyst. Examples of the reason for the reduced activity of the catalyst include the accumulation of a high-boiling point compound that may result from the hydrogenation reaction on the catalyst. In the regeneration treatment, the catalyst is presumably regenerated by bringing the hydrogen-containing gas into contact with the catalyst and thereby removing the high-boiling point compound through degradation and vaporization caused by hydrogenolysis. Thus, aside from hydrogen for use in the hydrogenation to be carried out for xylylenediamine production, additional hydrogen is required for hydrogenolysis in the regeneration treatment. This provides room for enhancing production efficiency by reducing the amount of the latter hydrogen used.

In this context, the present inventors have studied direct recycling of the hydrogen-containing gas used in the regeneration treatment (exhaust gas) and consequently found that the desired reaction results cannot be maintained. This is presumably because the exhaust gas described above contains organic compounds (typically, liquid organic compounds) such as benzene, and as a result of supplying the exhaust gas again into the reactor, these organic compounds are decomposed on the catalyst surface to elevate a catalyst layer temperature, adversely affecting catalyst performance.

In light of the above description, the present inventors have conducted further studies and consequently found that the catalyst can be regenerated while the amount of hydrogen used is reduced, by carrying out an operation of removing an organic compound in exhaust gas, and reusing the resulting gas as recycled gas, and as a result, the desired reaction results can be maintained with enhanced production efficiency.

As described above, the production method of the present embodiment, which adopts two stages of hydrogenation reaction and involves the treatment step between the first hydrogenation step and the second hydrogenation step, eliminates the need of changing hydrogenation conditions in the second hydrogenation reaction from those in the first hydrogenation step, can continuously produce xylylenediamine over a long period, and is also excellent in production efficiency.

In the treatment step, at least a part of the catalyst is preferably washed with a solvent before the supply of the hydrogen-containing gas G2 into the reactor. The washing thus performed tends to promote the regeneration treatment of the catalyst in the treatment step. From a similar viewpoint, a liquid component present in the reactor is preferably separated and removed before the washing of the catalyst.

In the present embodiment, the timing to interrupt the hydrogenation can be determined in consideration of, but not limited to, the composition of a reaction liquid (after removal of the solvent from the reaction liquid discharged from the reactor) and the state of the reactor. The timing can be determined on the basis of, for example, an event in which in the first hydrogenation step, the amount of 3-cyanobenzylamine in the reaction liquid reaches a predetermined amount or more, or the differential pressure between an entry and an exit of a catalyst layer in the reactor reaches a predetermined value or more.

In the treatment step, the contact temperature between the hydrogen-containing gas G2 and the catalyst that has undergone the first hydrogenation step is preferably 200°C to 500°C. The contact temperature of 200°C or higher tends to enhance the effects of the regeneration treatment. The contact temperature of 500°C or lower tends to be able to prevent deterioration in the catalyst caused by the temperature. The contact temperature in the treatment step means a temperature in a steady state after a process of elevation in catalyst temperature mentioned later, and can be identified as a catalyst layer temperature in the reactor. The contact temperature according to the present embodiment and the length of the treatment time are also determined in consideration of the type of the catalyst and the degree of reduction in activity. In particular, a longer treatment time is preferred for a marked degree of reduction in activity of the catalyst that has undergone the first hydrogenation step. In the present embodiment, the contact temperature of 200°C to 500°C is preferably maintained for 3 to 300 hours.

In the treatment step, the temperature elevation rate of the catalyst is preferably 40°C/min or less (including zero), more preferably 30°C/min or less, further preferably 20°C/min or less, while the hydrogen-containing gas G2 is brought into contact with the catalyst that has undergone the first hydrogenation step. In the present embodiment, the contact temperature is preferably 200 to 500°C, as mentioned above. Before the temperature range is attained, the hydrogen-containing gas G2 may be brought into contact with the catalyst at lower than 200°C. In this case as well, the temperature elevation rate of the catalyst is preferably controlled to 40°C/min or less.

In the treatment step, the supply rate of the hydrogen-containing gas G2 can be appropriately adjusted depending on the type of the catalyst, the history of use, and the catalyst temperature and is not particularly limited. The supply rate of the hydrogen-containing gas G2 in the treatment step is preferably 0.001 to 2000 NL/h (N: normal state (0°C, 1 atm), more preferably 0.001 to 1000 NL/h, per kg of the catalyst from the viewpoint of properly controlling the temperature elevation rate of the catalyst.

The hydrogen-containing gas G2 may contain an optional component other than hydrogen and may contain, for example, inert impurities that do not impair the regeneration of the catalyst. Examples of the impurities include, but are not particularly limited to, methane and nitrogen. The hydrogen pressure of the hydrogen-containing gas G2 is preferably 0.01 kPa to 30 MPa, more preferably 0.1 kPa to 15 MPa. In the case of setting the hydrogen partial pressure to a low value (e.g., 0.1 MPa or lower), use of inert dilution gas such as nitrogen is convenient and preferred.

In the treatment step, the contact temperature is preferably 200 to 500°C, and the following treatment (hereinafter, also referred to as "pretreatment") may be carried out before the temperature range is attained. Specifically, the hydrogen-containing gas G2 may be brought into contact with the catalyst that has undergone the first hydrogenation step at a temperature that falls within the range of 140 to 200°C and has an average temperature of 180°C or lower for 1 hour or longer. In this case, the catalyst tends to be more efficiently regenerated, and heat generation or gas generation ascribable to the regeneration treatment tends to be reduced.

The average temperature in the pretreatment refers to a time-average temperature in the pretreatment carried out at 140 to 200°C and is defined by a value determined by integrating temperatures with respect to a time, and dividing the obtained value by the length of the treatment time. In carrying out the pretreatment, the temperature may vary in the middle of the treatment in the range of 140 to 200°C. For example, a process of maintaining a constant temperature may be combined with a process of elevating or lowering the temperature. The treatment time of the pretreatment may be set to 1 to 20 hours.

In the pretreatment, the elevation rate of the catalyst temperature in supplying the hydrogen-containing gas G2 is preferably 40°C/min or less (including zero), more preferably 30°C/min or less, particularly preferably 20°C/min or less.

In the pretreatment, the supply rate of the hydrogen-containing gas G2 can be appropriately adjusted depending on the type of the catalyst, the history of use, and the catalyst temperature and is not particularly limited. The supply rate of the hydrogen-containing gas G2 in the pretreatment is preferably 0.001 to 2000 NL/h (N: normal state (0°C, 1 atm), more preferably 0.001 to 1000 NL/h, per kg of the catalyst from the viewpoint of properly controlling the temperature elevation rate of the catalyst.

The composition of the hydrogen-containing gas G2 at the contact temperature of 200 to 500°C may be the same as or different from that of the hydrogen-containing gas G2 in the pretreatment.

The recycled gas in the treatment step is obtained by removing at least a part of an organic compound from exhaust gas emitted from the reactor through the contact.

Examples of the operation for removing at least a part of the organic compound from the exhaust gas include, but are not particularly limited to, adsorption, absorption, and gas-liquid separation. The organic compound according to the present embodiment may typically include a liquid organic compound, as mentioned above. Therefore, a gas-liquid separator can be preferably adopted. Specifically, in the present embodiment, the recycled gas is preferably obtained by removing at least a part of the organic compound from the exhaust gas using a gas-liquid separator, from the viewpoint of enhancing the removal efficiency of the organic compound from the exhaust gas while avoiding excessive equipment burden. From the same viewpoint as above, in the present embodiment, the supply temperature of the exhaust gas to the gas-liquid separator is preferably 5°C to 80°C.

The gas-liquid separator is not particularly limited, and various known devices can be adopted.

The hydrogen-containing gas G2 in the treatment step is not particularly limited as long as the gas comprises recycled gas. The hydrogen-containing gas G2 can be adjusted to various compositions. In the present embodiment, the hydrogen concentration of the hydrogen-containing gas G2 is preferably 1 vol% or more from the viewpoint of enhancing the efficiency of the regeneration treatment of the catalyst. In the present embodiment, the upper limit value of the hydrogen concentration of the hydrogen-containing gas G2 is not particularly limited and may be, for example, 90 vol% or less.

In the present embodiment, the timing to terminate the treatment step can be determined in consideration of, but not limited to, the degree of removal of a high-boiling point compound capable of accumulating on the catalyst. The timing can be determined on the basis of, for example, an event in which in the treatment step, the hydrogen concentration of the hydrogen-containing gas G2 has become equivalent to the hydrogen concentration of the exhaust gas. Specifically, when the hydrogen concentration of the hydrogen-containing gas G2 becomes equivalent to the hydrogen concentration of the exhaust gas, this suggests that the high-boiling point compound capable of accumulating on the catalyst has already been sufficiently removed.

### (Second hydrogenation step)

In the second hydrogenation step, the hydrogenation is resumed in the presence of the catalyst that has undergone the treatment step. In the present embodiment, the catalyst undergoes the treatment step and is thereby reactivated. Therefore, the desired reaction results can be achieved in the second hydrogenation step, as in the first hydrogenation step. The conditions of the hydrogenation in the second hydrogenation step can be the same as the contents described in the first hydrogenation step.

Since the second hydrogenation step is a step subsequent to the treatment step, the catalyst may be regarded as being in a sufficiently reduced state. Thus, in the second hydrogenation step, the hydrogenation is preferably carried out by omitting the reduction treatment (activation treatment) which may be optionally carried out in the first hydrogenation step.

The production method of the present embodiment can comprise, as mentioned above, the first hydrogenation step, the treatment step, and the second hydrogenation step and may optionally comprise various additional steps.

In the production method of the present embodiment, an additional treatment step may be carried out under the same conditions as those of the treatment step, and then, an additional hydrogenation step may be carried out under the same conditions as those of the first hydrogenation step, as additional steps subsequent to the first hydrogenation step, the treatment step, and the second hydrogenation step, in order to further continue xylylenediamine over a long period. As a specific example thereof, the production method of the present embodiment may comprise, as additional steps subsequent to the first hydrogenation step, the treatment step, and the second hydrogenation step: an additional treatment step of interrupting the hydrogenation in the second hydrogenation step and supplying hydrogen-containing gas comprising the recycled gas mentioned above to the reactor to bring the catalyst that has undergone the second hydrogenation step into contact with the hydrogen-containing gas; and an additional hydrogenation step of resuming the hydrogenation in the presence of the catalyst that has undergone the additional treatment step. In the production method of the present embodiment, the additional treatment step and the additional hydrogenation step may be repetitively carried out one or more times in this order as additional steps subsequent to the first hydrogenation step, the treatment step, and the second hydrogenation step.

In the production method of the present embodiment, a purification step may be carried out in a purification tower or the like placed on the downstream side of the reactor exit, in order to enhance the purity of xylylenediamine, after the first hydrogenation step, the treatment step, and the second hydrogenation step or after the additional steps subsequent to the first hydrogenation step, the treatment step, and the second hydrogenation step. The purification step can be carried out by the adoption of various known conditions.

### Examples

Hereinafter, the present embodiment will be described in more detail with reference to Examples. However, the present embodiment is not limited by these Examples.

### [Example 1]

### (1) First hydrogenation step

A tubular vertical hydrogenation reactor (made of SUS304, inner diameter: 32 mmϕ) was charged with 180 g of a commercially available Raney cobalt catalyst having a cobalt content of 53% by mass, and ventilated with 10% hydrogen-containing gas at 320 NmL/min at 280°C for 16 hours to perform activation treatment. After the activation treatment, the system was cooled, and the pressure was kept constant at 8 MPaG by the press-in of hydrogen gas into the reactor. The catalyst layer temperature was maintained at 60°C by heating from the outside. While hydrogen gas (hydrogen-containing gas G1) was distributed at a flow rate of 118 NmL/min from the upper part of the reactor, a mixed liquid of isophthalonitrile and liquid ammonia (isophthalonitrile:liquid ammonia = 5:95 (mass ratio)) was distributed at 2.25 g/h to the catalyst to carry out hydrogenation reaction (reaction temperature: 60°C; the starting material dicyanobenzene was supplied at a rate of 0.0625 parts by mass/hr based on 100 parts by mass of the catalyst). After 48 hours from the start of the reaction, a reaction liquid discharged from the reactor was sampled, and this sample was heated to 80°C under normal pressure to remove the liquid ammonia. The composition of the reaction liquid after the removal of the liquid ammonia was 97.0% by mass of metaxylylenediamine and 0.08% by mass of 3-cyanobenzylamine, and no isophthalonitrile was detected. Then, the reaction was continued, and the concentration of the 3-cyanobenzylamine in the reaction liquid after the removal of the liquid ammonia was confirmed to reach 0.2% by mass.

### (2) Treatment step

The hydrogenation reaction was interrupted by stopping the supply of the mixed liquid of isophthalonitrile and liquid ammonia and the hydrogen gas because the concentration of the 3-cyanobenzylamine in the reaction liquid after the removal of the liquid ammonia reached 0.2% by mass, as described above. Then, the catalyst was washed by the liquid passing of liquid ammonia alone at 5.0 g/h for 1 hour. The catalyst layer temperature was decreased to 40°C or lower by cooling, and the pressure of the reactor was set to normal pressure. Then, the reactor was ventilated with 10% hydrogen-containing gas at 32 NmL/min. Exhaust gas emitted from the reactor was supplied to a gas-liquid separator placed on the downstream side of the reactor exit. A part of the exhaust gas routed through the gas-liquid separator was brought as recycled gas (recycled gas obtained by removing at least a part of an organic compound from the exhaust gas emitted from the reactor) back to the reactor entry at 288 NmL/min using a compressor so that the reactor was ventilated with the gas (hydrogen-containing gas G2: hydrogen concentration: 1 vol% or more and 10 vol% or less) at a total of 320 NmL/min. Subsequently, the reactor was heated to elevate the catalyst layer temperature to 280°C over 2 hours. Regeneration treatment was performed for a total of 16 hours including the temperature elevation time. Then, the regeneration treatment was completed by stopping the heating (contact time at the contact temperature of 280°C: 14 hours). The supply temperature of the gas to the gas-liquid separator during the regeneration treatment was 25°C. After the completion of the regeneration treatment, a liquid collected in the gas-liquid separator was examined. As a result, 13.6 g of benzene, 7.1 g of heptane, and 0.5 g of metaxylene were trapped. A total of 3.1 NL hydrogen gas was freshly used in the treatment step.

### (3) Second hydrogenation step

After the step (2), the hydrogenation reaction was resumed under the same reaction conditions as in the step (1). The composition of a reaction liquid after removal of the liquid ammonia obtained after 48 hours from the resumption of the hydrogenation reaction was 95.2% by mass of metaxylylenediamine and 0.12% by mass of 3-cyanobenzylamine, and no isophthalonitrile was detected. Thus, almost the same results as those of the step (1) were shown.

### [Comparative Example 1]

The steps (1) to (3) were carried out in the same manner as in Example 1 except that in the step (2), no gas-liquid separator was placed on the downstream side of the reactor. As a result, the composition of a reaction liquid after removal of the liquid ammonia obtained after 48 hours from the resumption of the hydrogenation reaction in the step (3) was 95.5% by mass of metaxylylenediamine, and no isophthalonitrile was detected, whereas the amount of 3-cyanobenzylamine was drastically increased to become 0.52% by mass. Thus, reduction in catalyst activity was confirmed.

### [Comparative Example 2]

The steps (1) to (3) were carried out in the same manner as in Example 1 except that in the step (2), no gas-liquid separator was placed on the downstream side of the reactor; and after the reactor was ventilated with 10% hydrogen-containing gas at 320 NmL/min, exhaust gas emitted from the reactor was not reused (the exhaust gas was not brought as recycled gas back to the reactor). As a result, the composition of a reaction liquid after removal of the liquid ammonia obtained after 48 hours from the resumption of the hydrogenation reaction in the step (3) was 96.9% by mass of metaxylylenediamine and 0.08% by mass of 3-cyanobenzylamine, and no isophthalonitrile was detected. Almost the same results as those of Example 1 were shown. However, a total of 30.7 NL of hydrogen gas was freshly used in the treatment step. Thus, a large amount of hydrogen was used as compared with Example 1.

The present application claims priority to the Japanese patent application filed on June 30, 2023 (Japanese Patent Application No. 2023-108290), the contents of which are incorporated herein by reference.

## Claims

1. A xylylenediamine production method comprising:
a first hydrogenation step of supplying dicyanobenzene and hydrogen-containing gas G1 in the presence of a catalyst having hydrogenation ability to a reactor to obtain xylylenediamine by hydrogenation of the dicyanobenzene;
a treatment step of interrupting the hydrogenation and supplying hydrogen-containing gas G2 comprising recycled gas to the reactor to bring the catalyst that has undergone the first hydrogenation step into contact with the hydrogen-containing gas G2; and
a second hydrogenation step of resuming the hydrogenation in the presence of the catalyst that has undergone the treatment step, wherein
the recycled gas is obtained by removing at least a part of an organic compound from exhaust gas emitted from the reactor through the contact.

2. The xylylenediamine production method according to claim 1, wherein the recycled gas is obtained by removing at least a part of the organic compound from the exhaust gas using a gas-liquid separator.

3. The xylylenediamine production method according to claim 2, wherein a supply temperature of the exhaust gas to the gas-liquid separator is 5°C to 80°C.

4. The xylylenediamine production method according to claim 1 or 2, wherein in the treatment step, a contact temperature between the hydrogen-containing gas G2 and the catalyst that has undergone the first hydrogenation step is 200°C to 500°C.

5. The xylylenediamine production method according to claim 1 or 2, wherein a hydrogen concentration of the hydrogen-containing gas G2 is 1 vol% or more.

6. The xylylenediamine production method according to claim 1 or 2, wherein in the treatment step, at least a part of the catalyst is washed with a solvent before the supply of the hydrogen-containing gas G2 to the reactor.

7. The xylylenediamine production method according to claim 1 or 2, wherein the reactor is a fixed-bed reactor.

8. The xylylenediamine production method according to claim 1 or 2, wherein the catalyst comprises nickel and/or cobalt.
